(19) Europäisches Patentamt
European Patent Office
Office européen des brevets

(11) EP 2 273 262 A2

(12) EUROPEAN PATENT APPLICATION
published in accordance with Art. 153(4) EPC

(43) Date of publication:
12.01.2011 Bulletin 2011/02

(51) Int Cl.:
*G01N 33/48* (2006.01)

(21) Application number: 09723771.3

(22) Date of filing: 20.01.2009

(86) International application number:
PCT/KR2009/000291

(87) International publication number:
WO 2009/119972 (01.10.2009 Gazette 2009/40)

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(30) Priority: 28.03.2008 KR 20080028871

(71) Applicant: Digital Genomics Inc.
Seoul 133-110 (KR)

(72) Inventor: LEE, Jae-Hoon
Seoul 131-767 (KR)

(74) Representative: Lang, Christian
LangRaible GbR
Patent- und Rechtsanwälte
Rosenheimerstrasse 139
81671 München (DE)

(54) HIGHLY SENSITIVE BIO-SENSOR, BIOCHIP INCLUDING SAME AND METHOD FOR MANUFACTURING SAME

(57) The present invention has a feature to form a biocompatible dielectric thin film on the surface of a metal electrode in a biosensor constructing a biochip. When using such a biocompatible dielectric thin film, this non-specific adsorption between a protein such as enzyme and a metal electrode can be prevented. Therefore, the present invention can escape a phenomenon that inhibits the reaction of a biomolecule due to the non-specific adsorption on the surface of a metal electrode. In addition, when the dielectric thin film having a high dielectric constant, as a biocompatible dielectric thin film, is made on the surface of a metal electrode, the sensitivity of an electrical detection according to the reaction of a biomolecule can be improved.

Fig. 2

## Description

[Technical Field]

The present invention relates to a biosensor that detects the presence and/or the reaction of a biomolecule in a high sensitivity by monitoring the change of an electrical property accurately, according to biological, biochemical or chemical reaction of a biomolecule, a biochip comprising the same and a method for manufacturing the same.

Particularly, the present invention relates to a biosensor that prevents non-specific adsorption toward on the surface of a metal electrode detecting a biomolecule electrically, a biochip comprising the same and a method for manufacturing the same.

More particularly, the present invention relates to a biosensor that prevents the non-specific adsorption onto the surface of a metal electrode detecting a biomolecule electrically by forming a dielectric thin film on the surface of the metal electrode and increases the sensitivity and the reproducibility of electrical detection for a biomolecule, a biochip comprising the same and a method for manufacturing the same.

[Background Art]

Biochip is a device that can analyze genetic information and protein information automatically in a large scale, or detect the presence and the function of a biomolecule easily and rapidly. This biochip is being actively applied for various fields including gene and protein researches, medicines, and agricultural, environmental and chemical industries, etc.

The biochip is classified broadly to genotyping chip, expression chip and microfluidics chip: the genotyping DNA chip is to detect the presence of a particular gene by using a probe; the expression DNA chip is to monitor the expression profiling of gene associated with a particular disease; and the microfluidics chip is to detect the presence and/or the reaction of a biomolecule within a sample including blood and urine. Presently, the genotyping DNA chip is commercialized and used widely in research areas and medical diagnosis areas.

Generally, the term microarray chip defines a chip that arrays hundreds to ten thousands kinds of genes or proteins mounting on a glass plate by using a microarray apparatus. Among these, DNA chip is to microarray oligonucleotides as probes on a glass plate in order to identify the presence of a particular gene with a fluorescence scanner.

The DNA chip is being utilized practically for research and diagnosis fields. Particularly, this chip is applied to elucidate the gene function including cellular metabolism, physiological phenomena and mutual relation between genes by using gene expression profiling and genotyping techniques, etc. The DNA chip is also used widely in diagnosis to examine a mechanism causing a particular disease such as cancer, prognostic diagnosis and action of drugs, to identify genetic information of microbes causing diseases, and to screen mutations, etc.

Diagnostic DNA chip has been developed in 1994 by Dr. Steve Fodor in Affymetrix Co. Ltd., and the first HIV gene chip started to be commercialized in a market. Nowadays, researches upon the diagnostic DNA chip are attempted actively in order to diagnose chronic diseases including HIV, rheumatism, autoimmune disease, chronic nephritis, atherosclerosis, atopic dermatitis and allergy, etc. Especially, recent studies upon DNA chips tend to develop chips for diagnostic use rather than chips for research use. Moreover contrasting to genotyping chips useful for diagnosing genotype, pathogen and virus, an approach on gene expression profiling capable of diagnosing various diseases including cancer and leukemia, is being accomplished.

Recently, microfluidics chip (Lab-on-a-chip) that can detect a lot of diseases coincidently from one trial and predict outbreak of diseases from genetic information of an individual by introducing IT and nano technologies, attracts attention. The microfluidics chip is also referred to as biochip. This chip is used to analyze a reaction profiling of various biomolecules within a chip, after a minute amount of an analytic target material (DNA, RNA, peptide, protein, etc.) is introduced into a chip chamber. This biochip is to detect the presence and/or the reaction of a biomolecule by monitoring changes of electrical property from an electrode installed in a chip, after being reacted with the biomolecule in a reaction chamber.

Such a biochip is highly applicable for medical diagnosis, because it can identify the presence and/or the reaction of a biomolecule more easily and rapidly by detecting electrical signals than any other DNA chips mentioned above.

For example, HPV DNA chip is a device that prepares HPV oligonucleotide probes and microarrays these probes on a glass plate in order to diagnose whether HPV, a pathogenic virus causing cervical cancer is positive or not. Nevertheless, it is impossible to directly diagnose the positive status of HPV, right after suspected sample is collected. That is to say, primers for amplifying HPV viral gene, labeled with fluorescence should be prepared in advance. Then, the collected sample should be amplified by performing a PCR, mounted onto an HPV DNA chip and monitored to examine a fluorescent signal with a fluorescence scanner. However, this system for detecting hybrids by using a DNA chip (laser-induced fluorescence) is inconvenient to be manipulated and spends time a lot. Therefore, this method has various problems and disadvantages. It needs high cost due to labeling a DNA sample with a fluorescent material and is not portable because of using an expensive fluorescence scanner.

In contrast, the biochip can identify the presence and/or the reaction of a biomolecule relatively easily and rapidly by detecting electrical signals. Particularly, the biochip can detect the presence and/or the reaction of a biomolecule (for example, DNA) by using electrical signals rather than fluorescent signals. More particularly, the biochip adopts a system for detecting changes electrically, in which the change of an impedance value (or a capacitance value) is monitored after reacting a receptor immobilized onto an electrode with a biomolecule, or the change of an impedance value (or a capacitance value) is monitored after reacting between biomolecules in a chip chamber.

For example, it is reported in the PCR process that dNTP should degrade to dNMP and diphosphate and the resulting dNMP is polymerized simultaneously from a primer complementary to a DNA template sequence so as to synthesize DNA. Accordingly, the impedance value within a PCR reagent increases as DNA concentration increases (See Korean Patent Laid-open NO. 10-2004-0042021). Therefore, it is possible to determine whether the PCR reaction is performed and a particular DNA sequence exists or not, when a PCR reaction chamber is manufactured with a biochip structure and the changes of an impedance value in a reagent are detected electrically on an electrode installed in such a biochip.

In order to sharply determine whether certain biomolecule exists or reacts within a reaction chamber by using a biochip, it is important to perform an electrical detection accurately with a sensing electrode. For such a reason, various biosensors constructing a biochip are being developed.

However, a biomolecule itself or a reaction product of a biomolecule can be adsorbed non-specifically toward on the surface of a metal electrode within a biosensor constructing a biochip. For example, in a silicon-based PCR reaction chip that incorporates a pattern of gold (Au) electrodes within a PCR reaction chamber, enzymes such as DNA polymerase are adsorbed non-specifically on the surface of gold (Au) electrodes in a biosensor.

As disclosed hitherto, it is known that proteins such as enzyme could be adsorbed toward on the metal electrode of a biosensor non-specifically because of a hydrophobic interaction between the surface of a metal electrode of a biosensor and the hydrophobic pocket of an enzyme in its protein structure. This non-specific adsorption of proteins may also occur, when a SAM (self-assembly-monolayer) is made on the surface of a metal electrode (especially, the surface of an electrode comprised of gold) due to -SH group (thiol group) of amino acids in a protein such as enzyme. Such a non-specific adsorption of proteins onto the surface of a metal electrode permits the formation of an electrical bi-layer on the surface of a metal electrode.

The electrical bi-layer that is formed on the surface of a metal electrode in a biosensor when proteins and the like are adsorbed non-specifically thereto, may cause two kinds of major problems.

First, when the proteins and the like are adsorbed on the surface of an electrode in a biosensor, the biological, biochemical or chemical reaction of a biomolecule is obstructed. In detail, in case of adsorbing DNA polymerase non-specifically onto the surface of a metal electrode within the above-mentioned PCR reaction chip, a PCR reaction is blocked and thus, a PCR product is not generated even if a target template DNA exists in a sample. Therefore, the biosensor electrode cannot determine the presence and/or the reaction of a target template DNA electrically.

Second, when the proteins and the like are adsorbed onto the surface of an electrode non-specifically to form an electrical bi-layer in a biosensor, electrical values detected in a biosensor can be fluctuated. Therefore, it is difficult to conduct a precise analysis with a biosensor and a biochip comprising the same.

Regarding these problems, conventional biochips as described in Korean Patent Laid-open No. 10-2004-0042021 have adopted a system for determining the reaction and the presence of a particular biomolecule by monitoring a change of impedance with sensing electrodes provided in a biochip after reacting a biomolecule. Accordingly, in order to guarantee the reliability of a biochip, it is important to measure the change of the impedance value accurately with sensing electrodes. Generally, impedance (Z) indicates the sum of resistance (R) as a real number portion and reactance (X) as an imaginary number portion (See following Mathematical formula 1), and the magnitude of impedance corresponds to a square root of resistance score (R) and reactance score (X) (See following Mathematical formula 2).

[Mathematical formula 1]

$$Z = R + jX$$
$$= R + j(X_L - X_C)$$
$$= R + j\left(\omega L - \frac{1}{\omega C}\right) \ [\Omega]$$

[Mathematical formula 2]

$$|Z| = \sqrt{R^2 + X^2}$$
$$= \sqrt{R^2 + \left(\omega L - \frac{1}{\omega C}\right)^2} \quad [\Omega]$$

Accordingly, the impedance (Z) value is made to have a correlation with the reactance (X). Also, the reactance (X) has a correlation with the capacitance (C) value because it is ωL-1/ωC. Therefore, the change of the capacitance (C) value varying according to biological, biochemical or chemical reactions, is reflected by the change of the impedance value, which enables the reaction and/or the presence of a biomolecule checked after its measurement. Finally, it is verified that this change of the capacitance value should change the impedance value in a biochip and influence upon the sensitivity of the biochip.

However, the change of a capacitance value in a biochip is not just directed by the reaction of a biomolecule. In detail, the change of values related to electrical property may also occur in sensing electrodes, when the non-specific adsorption on a metal electrode of a biosensor forms an electrical bi-layer as described above.

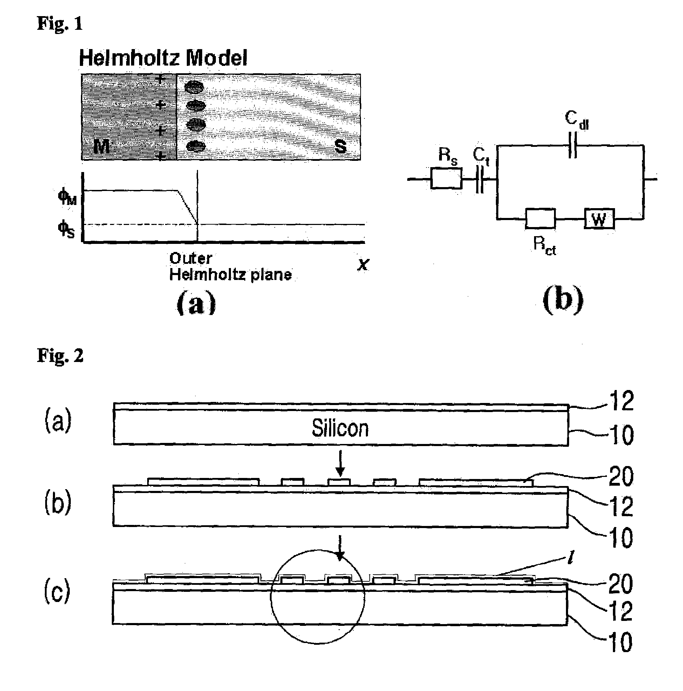

As confirmed in a Helmholtz model illustrated in FIG. 1(a), this non-specific adsorption of proteins and the like onto the surface of an electrode permits the formation of an electrical bi-layer. This can induce a drastic decrease of potential on the surface of an electrode. Such an electrical bi-layer can function for an equivalent circuit as illustrated in FIG. 1(b). As shown in FIG. 1(b), in addition to the change of a capacitance value induced by the reaction of a desired biomolecule for actual measurement, the change of a capacitance value ($C_{dl}$) of the electrical bi-layer also exists because of adsorbing proteins and the like non-specifically on the surface of an electrode.

Hence, when the change of a capacitance value is measured through sensing electrodes of a biochip, the total change of a capacitance value ($C_T$) in a biochip can include the change of a capacitance value ($C_{dl}$) of the electrical bi-layer due to the non-specific adsorption of proteins and the like on the surface of an electrode, and the change of a capacitance value ($C_t$) due to the reaction of a biomolecule between an imaginary electrode plate separated from the electrical bi-layer and another electrode (See following Mathematical formula 3).

[Mathematical formula 3]

$$C_T = \frac{1}{\frac{1}{C_t} + \frac{1}{C_{dl}}}$$

As demonstrated in the Mathematical formula 3, in case that the reciprocal number ($1/C_{dl}$) of a capacitance value ($C_{dl}$) of the electrical bi-layer generated by the non-specific adsorption of proteins and the like onto the surface of an electrode becomes larger, the reciprocal number ($1/C_t$) of a capacitance value ($C_t$) changed after reacting a biomolecule tends to be passed over by the above value ($1/C_{dl}$). In this case, the change of a capacitance value ($C_t$) caused by the reaction of a biomolecule does not influence the total change of a capacitance value ($C_T$). Furthermore, the reciprocal number ($1/C_{dl}$) of a capacitance value ($C_{dl}$) of the electrical bi-layer resulted from the non-specific adsorption of proteins and the like onto the surface of an electrode, even if it is not so large, could affect the total change of a capacitance value ($C_T$) either. Therefore, the non-specific adsorption of proteins and the like on the surface of an electrode causes a problem that the electrical detection from a sensing electrode in a biochip becomes inaccurate.

In order to solve above-mentioned disadvantages, the inventor has accomplished to design a biosensor that forms a dielectric thin film on the surface of a metal electrode detecting a biomolecule electrically to prevent the non-specific adsorption and to monitor the reaction of a biomolecule electrically in a high sensitivity, and a biochip comprising the same.

[Disclosure]

[Object]

The object of the present invention is to settle the problems of conventional methods mentioned above, which includes non-specific adsorption against electrodes in a biosensor, blocking of a biomolecule reaction caused by the same and change of values related to an electrical property in electrodes of a sensor.

Particularly, the object of the present invention is to provide a biosensor that prevents the non-specific adsorption onto the surface of a metal electrode detecting a biomolecule electrically, a biochip comprising the same and a method for manufacturing the same. For example, the object of the present invention is to prevent the non-specific adsorption of proteins and the like onto the surface of a metal electrode within a reaction chamber in which electrodes for detecting PCR products and DNAs electrically are formed by using a MEMS (micro-electro-mechanical-system) thin film technique.

More particularly, the object of the present invention is to provide a biosensor that forms a dielectric thin film on the surface of a metal electrode detecting a biomolecule electrically and to prevent the non-specific adsorption so as to enhance the sensitivity and the reproducibility of electrical detection for a biomolecule according to the reaction of a biomolecule, a biochip comprising the same and a method for manufacturing the same.

[Technical Solution]

The present invention has a constitutional feature to form a biocompatible dielectric thin film on the surface of a metal electrode in a biosensor constructing a biochip. When such a biocompatible dielectric thin film is utilized, the non-specific adsorption between a protein such as enzyme and a metal electrode as demonstrated above can be prevented.

In addition, when a dielectric thin film that has a high dielectric constant is formed on the surface of a metal electrode as a biocompatible dielectric thin film, the sensitivity of electrical detection according to the reaction of a biomolecule can be improved within the reaction chamber of a biochip. This will be explained clearly as follows.

In general, the capacitance is defined as the following Mathematical formula 4.

[Mathematical formula 4]

$$C = \frac{Q}{V} = \varepsilon \frac{A}{t}$$

A : area of electrode
T : interval between electrodes
ε: dielectric constant of material between electrodes

Particularly as defined in the above-mentioned Mathematical formula 4, in order to measure the capacitance value according to the reaction of a biomolecule electrically with reflecting its change exactly, the reciprocal number $(1/C_{dl})$ of the capacitance value $(C_{dl})$ due to the non-specific adsorption of proteins and the like onto the surface of an electrode should be smaller. In detail, the reciprocal number $(1/C_{dl})$ value approaches zero, when the $C_{dl}$ value is an infinite number. Therefore, the total change of the capacitance value $(C_T)$ can reflect almost all the capacitance change due to the reaction of a biomolecule and thus, increase the sensitivity and the reproducibility of the electrical detection.

Accordingly, when the surface of a metal electrode is deposited with a thin film having a high dielectric constant, the $C_{dl}$ value becomes larger and its reciprocal number $(1/C_{dl})$ is fixed at near zero. As a consequence, the detection sensitivity of a biomolecule can increase remarkably under buffer solutions or electrolytes of reaction solutions that have a much lower dielectric constant than that of the thin film. Hence, the non-specific adsorption against the metal surface cannot influence the total change of the capacitance value $(C_T)$.

Accordingly, one embodiment of the present invention has a feature to form a dielectric thin film that is biocompatible and has a high dielectric constant on the surface of a metal electrode in a biosensor constructing a biochip.

The dielectric thin film having a high dielectric constant used in the present invention can be any dielectric thin film that can form a thin film on the surface of a metal electrode through a semiconductor vapor deposition process and does not obstruct the reaction of a biomolecule. For example, the thin film may be silicon dioxide thin film, silicon nitride thin film, oxidized silicon nitride thin film, PSG thin film, BPSG thin film or $Ta_2O_5$ thin film. But, the present invention is not limited hereto and it is understood to those skilled in this art not to exclude a thin film having a high dielectric constant, if not inhibiting a biological reaction.

Theologically, it is preferable to increase the $C_{dl}$ value of electrical bi-layer on the metal surface. However, the dielectric thin film having a high dielectric constant tends to have the insulation property, when it becomes thicker on the surface of a metal electrode. Therefore, the thickness of the dielectric thin film having a high dielectric constant may affect the sensitivity of an electrode monitoring a biomolecule electrically.

Accordingly, the dielectric thin film that has a high dielectric constant and is formed on the surface of a sensing metal electrode in a biochip has preferably about no more than $1\mu m$ of thickness and more preferably, about no more than 50 nm. Within an acceptable range for a semiconductor vapor deposition method, it is better to make the film become thinner. In order to make the dielectric thin film on the surface of a metal electrode in a biochip through a vapor deposition, several methods including chemical vapor deposition (CVD), vacuum evaporation or sputtering and the like can be adopted.

The biosensor of the present invention has a feature to comprise a plate; an insulation film formed on the plate; one or more sensing metal electrodes that are formed on the insulation film, and detect an electrical change after biological, biochemical or chemical reaction of a biomolecule; and wherein a dielectric thin film is formed on the surface of the sensing metal electrode.

In addition, the method of manufacturing a biosensor in the present invention is comprised of the followings: preparing a plate; forming an insulation film on the plate; depositing a metal layer on the insulation film and patterning the metal layer using a photolithography process; etching the patterned metal layer to form a metal electrode; and forming a dielectric thin film on the surface of the metal electrode.

Further, the biochip of the present invention comprising the above-mentioned biosensor has a feature to comprise a first plate; an insulation film formed on the first plate; one or more sensing metal electrodes that are formed on the insulation film, and detect an electrical change after biological, biochemical or chemical reaction of a biomolecule; a second plate being placed in a predetermined distance from the first plate and being bound to the first plate so as to form a space of a reaction chamber; and wherein a dielectric thin film is formed on the surface of the sensing metal electrode.

In one embodiment of the biochip of the present invention, the second plate further comprises an insulation film formed on the second plate; and one or more sensing metal electrodes that are formed on the insulation film, and detect an electrical change after biological, biochemical or chemical reaction of a biomolecule.

In one embodiment of the present invention, the electrical change can be a change of impedance and/or a change of capacitance.

In one embodiment of the present invention, the dielectric thin film is preferably, a thin film comprised of a substance having a high dielectric constant. Preferably, the thin film comprised of a substance having a high dielectric constant can be at least one selected from a group consisting of silicon dioxide thin film, silicon nitride thin film, oxidized silicon nitride thin film, PSG thin film, BPSG thin film and $Ta_2O_5$ thin film. More preferably, the dielectric thin film comprised of a substance having a high dielectric constant can be silicon dioxide thin film or silicon nitride thin film.

In one embodiment of the present invention, the dielectric thin film has preferably about no more than $1\mu m$ of thickness and more preferably, about no more than 50 nm of thickness.

In one embodiment of the present invention, the metal electrode is preferably, made of gold, chrome, copper or aluminum.

In one embodiment of the present invention, "biomolecule" can be a nucleic acid composed of one or more nucleotides, a protein composed of one or more peptides, an amino acid, a glycolipid, or a low molecular weight compound, and preferably, antigen, DNA, RNA or PNA (peptide nucleic acid).

In one embodiment of the present invention, the plate can have a square, rectangular, or round shape, but the present invention is not limited hereto. The first plate and the second plate can be preferably, a silicon plate or a glass plate, but the plates can be composed of any one selected among fused silica, polystyrene, polymethylacrylate, polycarbonate, gold, silver, copper, or platinum. The first plate or the second plate is an N- type or a P-type silicon plate on which one or more metal electrodes are formed on a $SiO_2$ insulation layer, and performs electrical detection in order to identify the presence and/or the reaction of a biomolecule. The first plate or the second plate can be directly bound to the second plate or the first plate, respectively so as to form a space of a reaction chamber, with preventing metal electrodes of the second plate or metal electrodes of the first plate from contacting outer environment. The first plate and the second plate can be directly bound to each other, or indirectly bound to a glass wafer intervening between the plates.

[Advantageous Effects]

As illustrated and confirmed above, the biochip of the present invention has an advantageous effect that prevents the non-specific adsorption toward on the surface of an electrode detecting a biomolecule electrically so as to facilitate the reaction of a biomolecule within a biochip.

In addition, the present invention prevents the non-specific adsorption on the surface of a metal electrode detecting a biomolecule electrically by forming a dielectric thin film on the surface of the metal electrode so that the sensitivity and the reproducibility of the electrical detection are improved according to the reaction of a biomolecule.

Particularly, in the detection method for measuring an impedance as a resistance of alternating current (AC), the present invention allows the detection of a biological reaction in a high reproducibility and in a high sensitivity as well as prevents the non-specific adsorption effectively when detecting the biological reaction under an environmental condition of electrolytes or a biological buffer solution.

[Brief Description of Drawings]

The above and other objects, features and other advantages of the present invention will be more clearly understood to those skilled in this arts from the following detailed description taken in conjunction with the accompanying drawings, in which;

FIG. 1 is a schematic diagram of Helmholtz model and shows a drastic decrease of potentials on the surface of an electrode due to an electric bi-layer formed on the surface of an electrode.

FIG. 2 is a flow chart of the procedure in the method for preparing a biosensor in one embodiment of the present invention, which illustrates a step of forming a pattern of metal electrodes on a silicon plate and forming a dielectric thin film through a vapor deposition.

FIG. 3 is a sectional view of the electrode portion in the biosensor manufactured according to the procedure of FIG. 2 after being magnified.

FIG. 4 is a sectional view of a PCR reaction chip (200) in one embodiment of the present invention.

FIG. 5 is a flow chart of the procedure in one embodiment of the present invention, which illustrates steps of manufacturing a PCR reaction chip (200).

FIG. 6 is a comparison photograph of electrophoresis results in PCR reactions performed by using a PCR reaction chip (200) in one embodiment of the present invention and PCR reactions performed by using control PCR reaction chips that are not deposited with a dielectric thin film on the surface of a metal electrode through a vapor deposition (34 mm$^2$ of the surface area of an electrode and 10 mm$^2$ of the surface area of an electrode, respectively) .

[Mode for Invention]

Practical and presently preferred embodiments of the present invention are illustrated more clearly as shown in the following examples. However, it should be appreciated that those skilled in the art, on consideration of this disclosure, may make modifications and improvements within the spirit and scope of the present invention. References cited in the specification are incorporated into the present invention.

**Examples**

**Example 1: Construction of a biosensor**

The biosensor of the present invention is manufactured by using general procedures disclosed in this field, including a thin film formation technique of silicon dioxide, a photolithography technique, a light exposure patterning technique, a developing technique, a wet and/or dry etching technique, etc. Particularly, sensing metal electrodes are formed on a silicon plate by using a MEMS (micro-electro-mechanical-systems) and then, a silicon dioxide film or a silicon nitride film is deposited onto the surface of the metal electrode by using a CVD method.

The process for constructing a biosensor in one embodiment of the present invention will be described according to stages as follows.

**Example 1-1: Formation of a silicon plate and a metal electrode pattern**

Referring to FIG. 2, the process for manufacturing a biosensor in one embodiment of the present invention will be explained.

An N-type silicon wafer (10) having 500μm of thickness was thermally oxidized to form about 5,000Å of an SiO$_2$ layer (12) as an oxidized insulation layer (See the Step (a) of FIG. 2). Then, Ti/Au (300 Å/3,000Å) of a metal layer (20) was patterned to form a metal electrode by using a general photolithography process (See the Step (b) of FIG. 2).

**Example 1-2: Vapor deposition of dielectric thin film onto the surface of metal electrode**

The procedure for depositing a dielectric thin film of a silicon dioxide thin film or a silicon nitride thin film (*l*) through a vapor deposition onto the surface of a metal electrode (20) is conducted by using well-known vapor deposition methods and equipments related with a chemical vapor deposition.

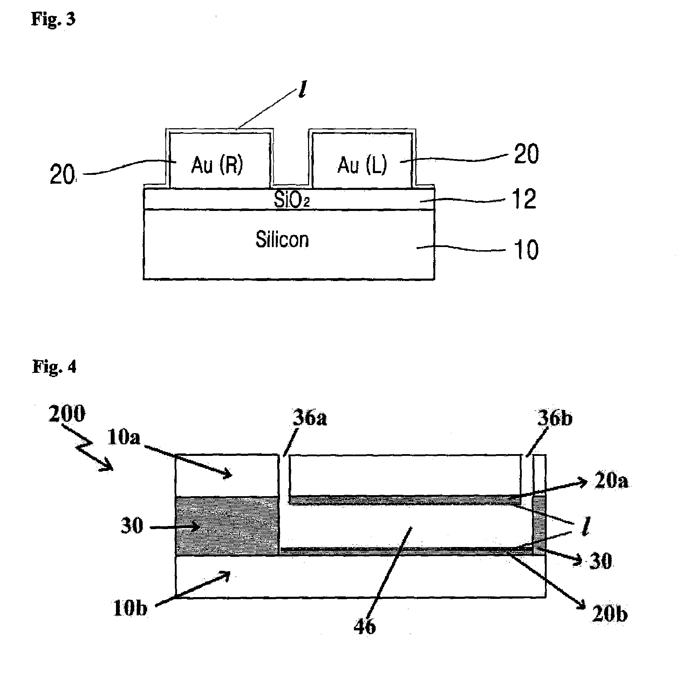

As illustrated in FIG. 2 (b), when metal electrodes (20) are formed onto a silicon plate (10) with an insulation film (12) intervening between them, a silicon dioxide thin film or a silicon nitride thin film (*l*) is deposited on the surface of a metal electrode (20) (See the (c) step of FIG. 2). FIG. 3 illustrates a metal electrode (20) that is deposited with a silicon dioxide thin film or a silicon nitride thin film (*l*) through a vapor deposition, after being magnified.

Particularly, general equipments for chemical vapor deposition usually used in this field are adopted in this example to deposit a dielectric thin film (*l*) of a silicon dioxide thin film or a silicon nitride thin film through a vapor deposition. The general equipments for chemical vapor deposition includes an LPCVD system (low pressure chemical vapor deposition system), a PECVD system (plasma enhanced CVD system), a P-5000 system and the like.

The chemical vapor deposition method using an LPCVD system is a method for performing a chemical vapor deposition under a low pressure. Advantageously, this system can deposit a film through a vapor deposition in a high purity and with a uniformity, but disadvantageously has a low rate of vapor deposition and a high temperature of vapor deposition. In addition, the chemical vapor deposition method using a PECVD (plasma enhanced CVD) system is a method for conducting a chemical vapor deposition by using plasma. This system is advantageous to have a high rate of vapor deposition and operate at a low temperature of vapor deposition, but disadvantageous to generate contaminants. Besides, a P-5000 system is a kind of PECVD system constituted in horizontally fixed type units and performs a chemical vapor deposition by using plasma.

In the present examples, a silicon dioxide thin film or a silicon nitride thin film (*l*) is made through a vapor deposition on the surface of a metal electrode (20) by using a PECVD system, an LPCVD system and a P-5000 system. In the present example, conditions of vapor deposition according to an apparatus of vapor deposition that are used to form a dielectric thin film (*l*) on the surface of a metal electrode (20) through a vapor deposition will be described as follows. In the meantime, it is understood naturally to those skilled in this arts that an ALD (atomic layer deposition) recently developed by modifying a CVD method could be adopted to manufacture a dielectric thin film on the surface of a metal electrode (20), even though not being described in this example.

## 1. PECVD system

### (1) Vapor deposition condition of silicon nitride thin film

(a) Gas flow rate: (unit: sccm)

5% $SiH_4/N_2$: 800 sccm
$NH_3$: 10 sccm
$N_2$: 1200 sccm

(b) Pressure : 580 mTorr
(c) Power source (RF Power): low frequency number (187kHz), 60W
(d) Depo. Rate: 160 Å/min
(e) Temperature (°C): 300°C

### (2) Vapor deposition condition of silicon dioxide thin film

(a) Gas flow rate: (unit: sccm)

5% $SiH_4/N_2$: 160 sccm
$N_2O$: 1500 sccm
$N_2$: 240 sccm

(b) Pressure: 550 mTorr
(c) Power source (RF Power): low frequency number (187kHz), 60W
(d) Depo. Rate: 340 Å/min
(e) Temperature (°C): 300°C

## 2. LPCVD system

**(1) Vapor deposition condition of silicon nitride thin film**

(a) Gas flow rate: (unit: sccm)

$SiH_2Cl_2$: 30 sccm
$NH_3$: 100 sccm

(b) Pressure: 300 mTorr
(c) Temperature (°C)
Front: 775°C
Center: 785°C
Rear: 795°C
(d) Depo. Rate: 35-45 Å/min

**(2) Vapor deposition condition of low stress silicon nitride thin film**

(a) Gas flow rate: (unit: sccm)
$SiH_2Cl_2$: 100 sccm
$NH_3$: 20 sccm
(b) Pressure: 140 mTorr
(c) Temperature (°C)
Front: 825°C
Center: 835°C
Rear: 845°C
(d) Depo. Rate: 35-45 Å/min

**3. P-5000 system**
**Vapor deposition condition of silicon dioxide thin film using TEOS oxide (Tetraethoxysilane oxide)**

(a) Gas flow rate: (unit: sccm)

TEOS source: 220 sccm
$O_2$: 220 sccm

(b) Pressure: 9 Torr
(c) Power source (RF Power)(W): 350W (at 13.56 MHz)
(d) Depo. Rate: 125 Å/min
(e) Temperature (°C): 390°C

The completed pattern of metal electrodes (20) in a biosensor is illustrated in FIG. 3. FIG. 3 illustrates a portion of the metal electrodes depicted in FIG. 2 (c) schematically after being magnified. As shown in FIG. 3, it is confirmed that a silicon dioxide thin film or a silicon nitride thin film (*l*) was thinly deposited through a vapor deposition on the surface of metal electrodes (20) formed on a silicon plate (10) of a biosensor in the present invention.

**Example 2: Construction of a PCR reaction chip**

The biosensor manufactured in Example 1 can be applied for various biochips. For example, it is applicable for a PCR reaction chip for PCR amplification reaction.
FIG. 4 illustrates a three-stepped PCR reaction chip in which a silicon plate (10a) forming an upper electrode (20a) and a silicon plate (10b) forming a lower electrode (20b) are bound to each other with a glass wafer intervening between the plates. The present invention does not limited hereto, but for example, it can be designed for a two-stepped PCR reaction chip in which paired silicon plates (10) are directly bound to each other. Further, it will be understood to those skilled in this arts that this biosensor is applicable for all known biochips, including a PCR reaction chip in which IDE electrodes are formed in a chamber, a DNA hybridization reaction chip and the like.
Hereinafter, the process for manufacturing a three-stepped PCR reaction chip (200) adopting a biosensor manufactured in Example 1 will be described according to stages as follows.
Above all, a silicon plate (10a)(see FIG. 4) on which an upper electrode (20a) is formed, and a silicon plate (10b)(see FIG. 4) on which a lower electrode (20b) is formed are manufactured by the procedures as illustrated in FIG.

5. After that, the upper and the lower silicon plates (10a, 10b) are indirectly bound to each other with a glass wafer (30) intervening between the plates to make a three-stepped PCR reaction chip (200) as depicted in FIG. 4.

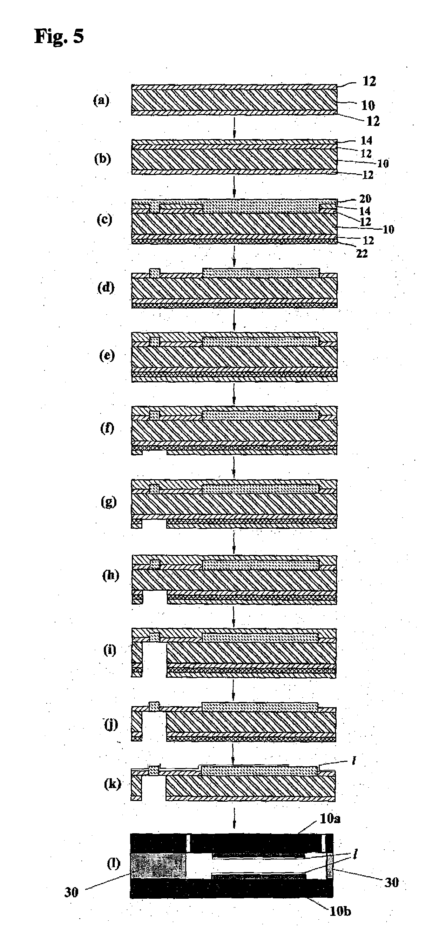

First, an N-type silicon wafer (10) having $300\mu m$ of thickness dopped with As was thermally oxidized to form about 6,500Å of an $SiO_2$ layer (12) as an oxidized film layer onto the upper and the lower sides of a silicon wafer (10) (See the Step (a) of FIG. 5). And then, the upper layer of an oxidized film (12) was coated with photoresist AZ 5214 (14) (See the Step (b) of FIG. 5). A mask (not shown) having a pattern of metal electrodes was mounted on the photoresist (14) and exposed with ultra violet light. Then, the silicon wafer (10) was immersed in a developing solution to be developed and treated with etching. On the upper side of the etched wafer (10), thin films of chrome (300Å) and gold (2,000Å) were deposited through a vapor deposition. On the lower side of the etched wafer (10), thin film (22) of aluminum (1,000Å) was deposited through a vapor deposition (See the (c) Step of FIG. 5). The vapor deposition of the thin films (20) of chrome and gold was performed by using a chemical vapor deposition (CVD), vacuum evaporation or sputtering. Then, the remaining photoresist layer (14) and a part of the thin film on the remaining photoresist layer (14) were removed by using a known lift-off process so as to make a metal electrode (20) (See the (d) Step of FIG. 5). After that, the upper and the lower sides of a silicon wafer (10) were coated with photoresist AZ4620 (See the (e) Step of FIG. 5) to form a pattern of photoresist on the thin film (22) of aluminum. Then, the thin film (22) of aluminum was treated with etching. Again, an oxidized insulation layer (12) and a silicon plate (10) were treated with etching (See the (f), (g), (h) and (i) Steps of FIG. 5). After etching, the remaining photoresist was removed. The thin film (22) of aluminum left was finally etched. By using a method as described in Example 1, the dielectric thin film (*l*) was deposited through a vapor deposition (See the (j) and (k) Steps of FIG. 5).

The upper silicon wafer (10a) and the lower silicon wafer (10b) manufactured by the above-mentioned process were indirectly bound to each other, with a glass wafer (30) ($1,000\mu m$) intervening between them by using an anodic bonding (See the (1) Step of FIG. 5C). In the glass wafer (30), a space of a reaction chamber (46) for a PCR chip is formed by using a sand blast proces. The bound silicon plates (10a, 10b and the glass wafer (30) were diced to manufacture a final PCR reaction chip (200) (See FIG. 4).

The anodic bonding is a method for binding a silicon plate and a glass plate, in which cationic ions (for example, $Na^+$ ion) present within a glass plate are allowed to move to the opposite direction with respect to the position where the silicon plate and the glass plate are earthed, to form hydrogen bonds on the surfaces of the silicon plate and the glass plate and to bind the two plates by applying hot heat and high electric field.

The PCR reaction chip (200) manufactured according to the present example has a structure that the upper electrode (20a) and the lower electrode (20b) face each other three-dimensionally.

In the PCR reaction chip (200), the upper metal electrode (20a) and the lower metal electrode (20b) are formed on the upper silicon plate (10a) and the lower silicon plate (10b), respectively. The surfaces of these metal electrodes (20a, 20b) are deposited with a silicon dioxide thin film or a silicon nitride thin film (*l*) through a vapor deposition. The upper silicon plate (10a) is placed in a predetermined distance from the lower silicon plate (10b), and bound to the lower silicon plate (10b) by a glass wafer (30) so as to form a space of a reaction chamber (46).

The space of the reaction chamber (46) is a space that accommodates a target sample and a PCR reaction solution (including DNA polymerase) in order to conduct a PCR reaction. Disadvantageously, the polymerase of the PCR reaction solution filled in the space of the reaction chamber (46) obstructs a PCR reaction or distorts electrical detection from a metal electrode (20) because the polymerase can be adsorbed non-specifically onto the surface of a metal electrode (20) in conventional methods. However, the PCR reaction chip (200) of the present invention can prevent the non-specific adsorption of the polymerase effectively since it forms a dielectric film on the surface of a metal electrode.

In the meantime, the upper silicon plate (10a) is perforated to have a fluid inlet opening (36a) in the direction of thickness and further perforated to have a fluid outlet opening (36b) in the direction of thickness on the opposite side of the fluid inlet opening (36a) (See FIG. 4). As illustrated in FIG. 4, the inlet opening (36a) and the space of the reaction chamber (46), and the outlet opening (36b) and the space of the reaction chamber (46) are communicated with each other to allow fluid to flow therethrough in the reaction chamber (46). Through the inlet opening (36a), the PCR reaction solution and the target sample are introduced into the reaction chamber (46), and the PCR reaction solution and the target sample are discharged from the reaction chamber (46) through the outlet opening (36b).

## Example 3: Analysis of PCR reaction by using a PCR reaction chip

The PCR reaction chip (200) manufactured in Example 2 was used to perform a PCR reaction, and control PCR reaction chips that are not deposited with a dielectric thin film on the surface of a metal electrode through a vapor deposition ($34\ mm^2$ of the surface area of an electrode and $10\ mm^2$ of the surface area of an electrode, respectively) were also utilized to perform a PCR reaction.

The PCR reaction is performed with the PCR reaction chip (200) of the present invention and control PCR reaction chips ($34\ mm^2$ of the surface area of an electrode and $10\ mm^2$ of the surface area of an electrode, respectively) by using a Promega PCR Core System II PCR Kit (PCR reaction solution commercially available in order to perform a PCR easily; a product of Promega Co. Ltd., USA) and DNA template. The PCR reaction was repeated with 25 to 35 cycles as recommended by Promega, while adjusting time and temperature in the PCR reaction during each PCR cycle.

In addition, the PCR reaction was performd according to two kinds of PCR reaction conditions. Under the PCR condition 1, the concentration of DNA template was adjusted to 0.06 ng/$\mu$l and the concentration of DNA polymerase was adjusted to 0.1 U/$\mu$l in order to perform the PCR reaction. Under the PCR condition 2, the concentration of DNA template was adjusted to 0.06 ng/$\mu$l and the concentration of DNA polymerase was adjusted to 0.025 U/$\mu$l in order to perform the PCR reaction (See Table 1).

**Table 1: PCR Reaction Conditions**

|  | PCR condition 1 | PCR condition 2 |
|---|---|---|
| DNA template | 0.06 ng/$\mu$l | 0.06 ng/$\mu$l |
| polymerase | 0.1 U/$\mu$l | 0.025 U/$\mu$l |

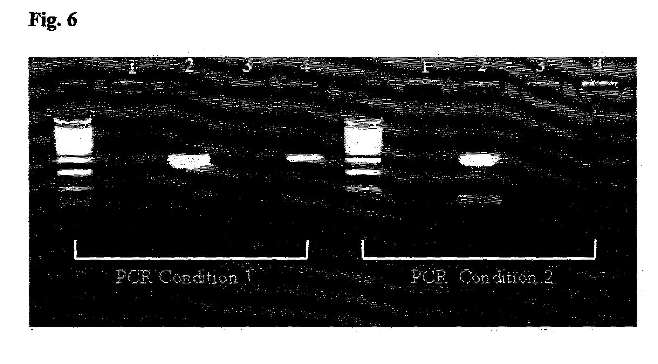

After performing PCR reactions by using the PCR reaction chip (200) manufactured in Example 2, the electrophoresis result was compared with that obtained from performing PCR reactions by using control PCR reaction chips that are not deposited with a dielectric thin film on the surface of a metal electrode through a vapor deposition (having 34 mm$^2$ of the surface area of an electrode and 10 mm$^2$ of the surface area of an electrode, respectively) (See FIG. 6).

As illustrated in FIG. 6, in case that DNA template is excluded in NO. 1 line, no band was observed because any PCR product was not generated. In case that PCR reactions are performed by using the PCR reaction chip (200) of the present invention (NO. 2 line), clear bands were observed under both the PCR condition 1 and the PCR condition 2. In contrast, when performing PCR reactions by using one control PCR reaction chip (having 34 mm$^2$ of the surface area of an electrode) (NO. 3 line), no band was observed under both the PCR condition 1 and the PCR condition 2. Besides, when performing PCR reactions by using the other control PCR reaction chip (having 10 mm$^2$ of the surface area of an electrode) (NO. 4 line), no band was observed under the PCR condition 2 and a weak band was detected under the PCR condition 1 increasing the concentration of DNA polymerase.

Considering overall results, it is identified that in case of control PCR reaction chips without forming a dielectric thin film on the surface of a metal electrode, PCR polymerization is obstructed due to the non-specific adsorption of proteins on the surface of a metal electrode. In contrast, in case of the PCR reaction chip (200) of the present invention, PCR reactions are proceeded actively to generate PCR products since the non-specific adsorption of proteins does not occur on the surface of a metal electrode.

Furthermore, in order to obtain desirable data, the impedance value between electrodes (20) is measured by applying alternating current (AC) to the metal electrode (20) of the biosensor in the PCR reaction chip of the present invention. Because the dielectric thin film such as a silicon dioxide thin film or a silicon nitride thin film is deposited on the surface of a metal electrode in the PCR reaction chip (200) of one embodiment of the present invention, it is preferable to measure the impedance value between electrodes (20) by applying alternating current (AC).

Therefore, it is identified that the biosensor of the present invention should prevent the non-specific adsorption of proteins on the surface of metal so as to perform the reaction of a biomolecule actively. Furthermore, this can improve the sensitivity of electrical detection and the reproducibility of detection according to the reaction of a biomolecule.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

**Claims**

1. A biosensor for detecting an electrical change in a reaction solution according to biological, biochemical or chemical reaction of a biomolecule comprising:

   a plate;
   an insulation film formed on the plate;
   one or more sensing metal electrodes that are formed on the insulation film, and detect an electrical change after biological, biochemical or chemical reaction of a biomolecule; and
   wherein a dielectric thin film is formed on the surface of the sensing metal electrode.

2. The biosensor as claimed in claim 1, wherein the dielectric thin film is a thin film comprised of a substance having a high dielectric constant.

3. The biosensor as claimed in claim 1, wherein the dielectric thin film is at least one selected from a group consisting of silicon dioxide thin film, silicon nitride thin film, oxidized silicon nitride thin film, PSG thin film, BPSG thin film and $Ta_2 O_5$ thin film.

4. The biosensor as claimed in claim 1, wherein the dielectric thin film is a silicon dioxide thin film or a silicon nitride thin film.

5. The biosensor as claimed in any one of claim 1 to 4, wherein the dielectric thin film has no more than 1$\mu$m of thickness.

6. The biosensor as claimed in any one of claim 1 to 4, wherein the electrical change is a change of impedance or a change of capacitance.

7. A method of manufacturing a biosensor that detects an electrical change in a reaction solution according to biological, biochemical or chemical reaction of a biomolecule comprising:

preparing a plate;
forming an insulation film on the plate;
depositing a metal layer on the insulation film and patterning the metal layer using a photolithography process;
etching the patterned metal layer to form a metal electrode; and
forming a dielectric thin film on the surface of the metal electrode.

8. The method of manufacturing a biosensor as claimed in claim 7, wherein the dielectric thin film is formed by depositing a substance having a high dielectric constant through a vapor deposition.

9. The method of manufacturing a biosensor as claimed in claim 7, wherein the dielectric thin film is formed by depositing at least one selected from a group consisting of silicon dioxide thin film, silicon nitride thin film, oxidized silicon nitride thin film, PSG thin film, BPSG thin film and $Ta_2 O_5$ thin film through a vapor deposition.

10. The method of manufacturing a biosensor as claimed in claim 7, wherein the dielectric thin film is formed by depositing a silicon dioxide thin film or a silicon nitride thin film through a vapor deposition.

11. The method of manufacturing a biosensor as claimed in any one of claim 7 to 10, wherein the dielectric thin film is formed to have no more than 1$\mu$m of thickness.

12. A biochip for detecting an electrical change in a reaction solution within a reaction chamber according to biological, biochemical or chemical reaction of a biomolecule comprising:

a first plate;
an insulation film formed on the first plate;
one or more sensing metal electrodes that are formed on the insulation film, and detect an electrical change after biological, biochemical or chemical reaction of a biomolecule;
a second plate being placed in a predetermined distance from the first plate and being bound to the first plate so as to form a space of a reaction chamber; and
wherein a dielectric thin film is formed on the surface of the sensing metal electrode.

13. The biochip as claimed in claim 12, wherein the dielectric thin film is a thin film comprised of a substance having a high dielectric constant.

14. The biochip as claimed in claim 12, wherein the dielectric thin film is at least one selected from a group consisting of silicon dioxide thin film, silicon nitride thin film, oxidized silicon nitride thin film, PSG thin film, BPSG thin film and $Ta_2 O_5$ thin film.

15. The biochip as claimed in claim 12, wherein the dielectric thin film is a silicon dioxide thin film or a silicon nitride thin film.

16. The biochip as claimed in any one of claim 12 to 15, wherein the dielectric thin film has no more than 1$\mu$m of thickness.

17. The biochip as claimed in any one of claim 12 to 15, wherein the second plate further comprises one or more sensing metal electrodes that detect an electrical change according to biological, biochemical or chemical reaction of a biomolecule.

18. The biochip as claimed in any one of claim 12 to 15, wherein the electrical change is a change of impedance or a change of capacitance.

**Fig. 1**

## Helmholtz Model

**(a)**

**(b)**

**Fig. 2**

Fig. 3

20

*l*

Au (R)     Au (L)     20

SiO₂     12

Silicon     10

Fig. 4

200

10a     36a     36b

30     20a

*l*

30

10b     20b

46

**Fig. 5**

(a)
12
10
12

(b)
14
12
10
12

(c)
20
14
12
10
12
22

(d)

(e)

(f)

(g)

(h)

(i)

(j)

(k)
*l*

(l)
10a
*l*
30
30
10b

**Fig. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020040042021